# EUROPEAN PATENT SPECIFICATION

(11) **EP 1 148 891 B1**
(45) Date of publication and mention of the grant of the patent: **17.03.2004**
(21) Application number: 99921347.3
(22) Date of filing: 22.04.1999
(51) Int. Cl.: A61L 2/08

(54) **Method of packaging an oxidation sensitive medicinal substance**
Packungsverfahren für Oxidationsempfindliche Medikamente
Procédé d'emballage pour médicaments sensibles a l'oxydation

(30) Priority: 19.01.1999 US 116360 P
(43) Date of publication of application: 31.10.2001
(73) Proprietor: PHARMACIA & UPJOHN COMPANY, Kalamazoo, Michigan 49001 (US)
(72) Inventor: MALLORY, Christopher, S., Kalamazoo, MI 49004 (US); BRITTEN, Nancy, J., Portage, MI 49024 (US); HAHN, David, A., Kalamazoo, MI 49008 (US); RE, Robert, G., Portage, MI 49024 (US); PYRET, Thomas, W., Portage, MI 49024 (US); SCHAPAUGH, Randal, L., Battle Creek, MI 49014 (US)
(74) Representative: Perry, Robert Edward
(86) International application number: PCT/US1999/006549
(87) International publication number: WO 2000/043049

(56) References cited:
- EP-A- 0 556 547
- US-A- 3 940 325
- US-A- 4 569 736

## Description

### Technical Field

The invention relates generally to sterilized packaging and, in particular, the invention relates to the use of polyethylene in the packaging of product materials for use with gamma-irradiation sterilization.

### Background Information

Gamma irradiation is frequently utilized as a sterilization technique for food, medical devices and medicinal products, as well as their respective packaging, i.e., some form of container. This is particularly true in the case of plastics, where sterilization techniques requiring heat may exceed the softening, or even melting, point of the plastic. Other sterilization techniques using aqueous or gaseous sterilants may also be unsatisfactory, due to contamination concerns or the like.

Gamma irradiation is usually carried out in one of two ways. A first method is to locate an article by a radiation source, typically a radioactive isotope of cobalt or cesium. The radiation source is commonly housed in steel casings, which are kept in a pool of water to absorb the gamma radiation while not in use. Articles to be irradiated are located near the pool of water and receive gamma irradiation when the casings are lifted out of the pool. A variation on this method is to pass the article by the radiation source using a conveyor.

A second method is to focus a beam of radiation directly on an article. This sterilizer unit typically contains a housing for containing the radiation source, a focusing ring for concentrating the radiation to a more localized region, and a beam outlet. Articles for sterilization are passed through the focused radiation, typically using a conveyor. In any irradiation method, radiation exposure is determined by the intensity of the radiation source or beam, and the length of exposure.

While gamma irradiation has its advantages over other forms of sterilization, it also has detriments. Irradiation of plastics results in energy transfer that is nonspecific both spatially and molecularly in the polymer. Two major chemical reactions occur as a result of this energy transfer: 1) crosslinking of the polymeric chains and 2) scission or the breaking of bonds resulting in creation of free radicals. BioMedical Polymers, Metals, and Composites, ch. 44, pp. 1001-18, "Ionizing Radiation's Effects on Selected Biomedical Polymers," Skiens, W.E. and Williams, J.L. (Technomic Publishing Co., 1983) (hereinafter "Ionizing Radiation"). Both of these reactions may occur simultaneously. and the predominating reaction dictates whether the polymer is degraded (scission) or increases in molecular weight due to polymerization (crosslinking).

Degradation products of the radical-induced reactions resulting from scission can consist of low molecular weight compounds (including the evolution of gases), unsaturation sites in the polymer chain (often the cause of discoloration), and peroxy species (which can abstract hydrogen to form hydroperoxides) in the presence of oxygen. Radicals resulting from irradiation can be long-lived and result in post-irradiation effects. These radicals can be trapped in the irradiated polymers and react over extended periods of time; the reaction rate depends on the reactivity of the sample and the mass transfer characteristics of the system. Oxidative reactions normally lead to scission, and cause deterioration in mechanical properties of the polymer. Free radicals produced by irradiation in oxygen-containing ambients, e.g., air, are often rapidly converted into peroxidic radicals.

Additives are often utilized to reduce the damaging effects of irradiation of the polymer. These types of additives are often called antirads. The antirads can either directly reduce damage by absorbing radiation and preventing interaction with the polymer, or indirectly reduce the effects of the damage by readily combining with the radiation-generated free radicals in the polymer. Antirads often also act as antioxidants.

The effects of gamma irradiation on polymers have been extensively studied. *See*, *e*.*g*., Thayer, Donald W., Chemical Changes in Food Packaging Resulting from Ionizing Irradiation, *Food and Packaging Interactions,* ch. 15 (1988) (hereinafter "Chemical Changes"); Killoran, John J., Chemical and Physical Changes in Food Packaging Materials Exposed to Ionizing Radiation, *Radiation Res. Rev.,* vol. 3, pp. 369-388 (1972) (hereinafter "Chemical and Physical Changes"); Ionizing Radiation. Killoran notes that the radiation stability of plastic films may be related to the total quantity of gaseous products evolved as a result of the ionizing radiation treatment. Chemical and Physical Changes, p. 376-77. As noted above, the evolution of gaseous products is an indicator of degradation by scission. Killoran further notes that research ranks plastic films in order of decreasing radiation stability, based on this scission-related criteria, as polyethylene terephthalate > polystyrene > polyiminoundecyl > poly(vinylidene chloride-vinyl chloride) > polyethylene. *Id*. at 377.

Of major concern to the pharmaceutical industry is the oxidative degradation of aqueous and oil-based formulations packaged in gamma-irradiation sterilized plastic containers. For many medicinal products packaged in plastic containers, either pre- or post-filling sterilization may be required. The free radicals produced as a result of scission during the irradiation of the polymeric packaging often lead to oxidative degradation of the medicinal product in contact with the polymer. Oxidative degradation of a medicinal product can result in lower potency of the active ingredient, reduced formulation efficacy, higher levels of impurities, unacceptable formulation physical properties, shorter product shelf life, and subsequent monetary losses related to reduced shelf life.

Although product safety is necessary for both foods and pharmaceuticals, the requirements for an irradiated packaging material for pharmaceuticals are more stringent than those for irradiated packaging materials for the food industry. Oxidative processes which can be tolerated or ignored in food applications may be unacceptable in the pharmaceutical industry, since food industry product concerns are primarily qualitative and subjective (organoleptic, i.e., the food's palatability, flavor, consistency, color, odor, etc.) while pharmaceutical industry product concerns are quantitative and objective.

The criteria for packaging materials for foods that will be irradiated consist primarily of 1) no significant negative change in any of the packaging material's important physical/mechanical characteristics (which may include toughness, tensile strength, tear resistance, hardness/pliability, resistance to solvents/light/humidity/etc.) and 2) that the packaging material not contaminate the food with irradiation-produced compounds.

"Safe for use after irradiation" is the primary regulatory criterion for packaging materials proposed for use in radiation-treated food stuffs. *See, e.g.,* 21 CFR 179.45 (U.S., 1998), Packaging Materials for Use During the Irradiation of Prepackaged Foods The requirements for pharmaceutical products are that they be safe, efficacious, and consistently possess known, measurable or quantifiable characteristics - such as potency, strength, and purity. These requirements are currently mandated by various laws.

Even minor changes to the pharmaceutical's physical, chemical, or biological properties (as may be caused by or initiated by contact with irradiated packaging materials) can and often do render the medicinal product unfit or unsafe for its intended use. For example, irradiated packaging for pharmaceuticals must not promote even minimal (less then 10% for antibiotics) active ingredient potency loss over the shelf life of the product which is often two to five years. Accordingly, it is not reasonable nor prudent to assume that simply because a material is acceptable for packaging irradiated food stuffs, it will be an acceptable packaging material for a pharmaceutical product over the entire shelf life of the pharmaceutical.

### Summary of the Invention

The above-mentioned problems with polymer irradiation and packaging of product materials and other problems are addressed by the method of the invention according to claim 1. For simplicity, product materials may be referred to simply as materials. Preferred embodiments of the invention are described by the dependent claims.

Stability studies of antibiotics packaged in gamma-irradiation sterilized polymer packaging materials indicate results inconsistent with expectations. Predictions based upon radiation stability of various polymers suggest that polyethylene would be inferior to several polymers in protecting oxidation-sensitive materials from oxidative degradation subsequent to gamma irradiation, that is, polyethylene would be expected to induce increased levels of oxidative degradation over various other polymers. However, the studies disclosed herein reveal that some classes of polyethylene are unexpectedly superior in their ability to protect materials from oxidative degradation subsequent to gamma irradiation.

Skiens and Williams teach that considerable carbon-carbon bond cleavage occurs in polyethylene upon irradiation. Ionizing Radiation, p. 1006. As oxidative degradation is generally related to free radicals resulting from scission, polyethylene would be expected to offer only marginal protection against oxidative degradation due to the considerable scission resulting from gamma irradiation. Accordingly, the ability of polyethylene to protect materials against oxidative degradation, as disclosed herein, is greater than expected.

The invention is applicable to oxidation-sensitive medicinal substances requiring gamma irradiation or gamma-irradiated packaging for storage, transport or use. Medicinal substances are used to prevent or treat disease, injury or pain. Medicinal substances may have human or animal applications. Accordingly, pharmaceuticals and veterinary products are suitable for use with the invention.

As used herein, a material is sensitive to oxidative degradation, or is oxidation sensitive, if the material suffers low potency of an active ingredient, reduced formulation efficacy, higher levels of impurities unacceptable formulation physical properties, shorter product shelf life or monetary loss as a result of contact with irradiation-induced peroxidic radicals. Primary examples include anti-infectives such as antibiotics, anti-fungals and anti-virals. However, oxidation-sensitive drugs in all classical pharmaceutical categories are well known. These categories include, but are not limited to, anti-histamines, laxatives, vitamins, decongestants, gastrointestinal sedatives, antacids, anti-inflammatory substances; anti-manics, coronary vasodilators, peripheral vasodilators, cerebral vasodilators, psychotropics, stimulants, anti-diarrheal preparations, anti-anginal drugs, vasoconstrictors, anti-coagulants, anti-thrombotic drugs, analgesics, anti-pyretics, hypnotics, sedatives, anti-emetics, growth promoters, anti-nauseants, anti-convulsants, neuro-muscular drugs, hyper and hypoglycemic agents, thyroid and anti-thyroid preparations, diuretics, cytotoxic compounds, ophthalmics, antispasmodics, uterine relaxants, anti-obesity drugs, anthelmintics, hormones, vaccines, mineral and nutritional additives and more.

Another category of materials of special interest is the genetically-engineered biopharmaceuticals which have special packaging needs to protect them from oxidative degradation.

Within the antibiotics family noted above, the classes of cephalosporins, lincosamides, quinolones, oxazolidinones, tetracyclines, penicillin and penicillin derivatives are of special interest, although almost all antibiotics are considered to be oxidation sensitive. In particular, the invention is applicable for use with pirlimycin, ceftiofur, lincomycin, neomycin, penicillin G and novobiocin.

In addition to oxidation-sensitive medicinal active ingredients, other non-active constituents of pharmaceutical formulations (products), such as vehicles and excipients may undergo oxidative degradation upon exposure to irradiated packaging materials. Oxidative degradation of a vehicle and/or excipient in a pharmaceutical formulation, even if the drug itself does not oxidize, could produce formulations with unacceptable characteristics before the end of the formulation's shelf life. Such unacceptable properties could include poor suspension resuspendability, difficult formulation syringability, objectionable product odor, color or taste, and reduction of preservative action. Formulations having an oxidation-sensitive constituent will be considered oxidation sensitive as a whole.

The polyethylene-containing packaging materials utilized in various embodiments of the invention may contain one or more additives incorporated with the polyethylene. Such additives include, but are not limited to, mold-release agents, stabilizers, antioxidants, antirads, compounding agents, lubricants, slip agents, colorants and copolymers. Preferably, polyethylene is the predominant constituent in the packaging material. While other polymers may be added to the polyethylene as copolymers without departing from the scope of the invention, it is recognized that such additions may result in higher levels of induced oxidative degradation of product material due to the inclusion of such other polymers in the packaging material.

As used herein, a packaging material is in contact with a product material if it is in direct and physical contact with the product material. A packaging material is also in contact with a product material if irradiation-induced radicals from the packaging material are free to migrate to a surface of the product material, e.g., through a semi-permeable member.

### Brief Description of the Drawings

Figure 1 is an elevation view of a radiation source and a packaged substance.
Figure 2 is a plan and elevation view of a packaging.
Figure 3 is a plan and elevation view of a packaging
Figure 4 is an elevation view of a packaging.
Figure 5 is a sectional view of a composite container.
Figure 6 is a sectional view of another embodiment of a composite container.

### Detailed Description of the Invention

In the following detailed description of the invention, reference is made to the accompanying drawings which form a part hereof, and in which is shown, by way of illustration, specific embodiments in which the invention may be practiced. In the drawings, like numerals describe substantially similar components throughout the several views. These embodiments are described in sufficient detail to enable those skilled in the art to practice the invention. Other embodiments may be utilized and structural, logical, and other changes may be made without departing from the scope of the invention. The following detailed description is, therefore, not to be taken in a limiting sense, and the scope of the invention is defined only by the appended claims.

Polyethylene is commonly divided into classes based on its density. Classes commonly used include low density polyethylene (LDPE), medium density polyethylene (MDPE) and high density polyethylene (HDPE). This list of classifications should not be considered as a standard or a complete list of classifications. It is provided merely to focus the following narrative.

Given these rather loose classifications, polymer characteristics vary among multiple producers of a given class of polyethylene, or among multiple grades of a given class by one producer. Furthermore, what one producer terms LDPE might be considered MDPE by another producer. Despite these variations, some generalizations can be made.

Table 1 lists typical values for some physical, mechanical and thermal properties of LDPE as used herein.

**Table 1**

| Typical Properties of Low Density Polyethylene | | |
|---|---|---|
| **Property** | **Value** | **Range / Comments** |
| Density, g/cc | 0.91 | 0.910-0.925 g/cc |
| Hardness, Shore D | 44 | 41-46 Shore D |
| Tensile Strength, Yield, MPa | 10 | 4-16 MPa; ASTM D638 |
| Tensile Strength, Ultimate, MPa | 25 | 7-40 MPa |
| Modulus of Elasticity, GPa | 0.2 | 0.07-0.3 GPa; In Tension; ASTM D638 |
| Flexural Modulus, GPa | 0.4 | 0-0.7 GPa; ASTM D790 |
| Coefficient of Thermal Expansion, linear 20°C, µm/m-°C | 30 | 20-40 µm/m° 1C; ASTM D696 |
| Melting Point, °C | 115 | |

Table 2 lists typical values for some physical, mechanical and thermal properties of MDPE as used herein.

**Table 2**

| Typical Properties of Medium Density Polyethylene | | |
|---|---|---|
| **Property** | **Value** | **Range / Comments** |
| Density, g/cc | 0.93 | 0.926-0.940 g/cc |
| Hardness, Shore D | 55 | 50-60 Shore D |
| Tensile Strength, Yield. MPa | 16 | 8-24 MPa; ASTM D638 |
| Tensile Strength, Ultimate, MPa | 25 | 8.3-45 MPa |
| Modulus of Elasticity, GPa | 0.3 | 0.14-0.41 GPa; In Tension; ASTM D638 |
| Flexural Modulus, GPa | 0.7 | ASTM D790 |
| Coefficient of Thermal Expansion, linear 20°C, µm/m-°C | 27 | ASTM D696 |
| Melting Point, °C | 125 | |

Table 3 lists typical values for some physical, mechanical and thermal properties of HDPE as used herein. HDPE may further include higher density polyethylenes beyond the density range of 0.941-0.97 g/cc listed here as typical.

**Table 3**

| Typical Properties of High Density Polyethylene | | |
|---|---|---|
| **Property** | **Value** | **Range / Comments** |
| Density. g/cc | 0.95 | 0.941-0.97 g/cc |
| Hardness, Shore D | 65 | 60-70 Shore D |
| Tensile Strength, Yield, MPa | 30 | 20-40 MPa; ASTM D638 |
| Tensile Strength, Ultimate, MPa | 50 | 20-70 MPa |
| Modulus of Elasticity, GPa | 0.8 | 0.4-1.2 GPa; In Tension; ASTM D638 |
| Flexural Modulus, GPa | 1.4 | 0.7-2 GPa; ASTM D790 |
| Coefficient of Thermal Expansion, linear 201C, µm/m-°C | 22 | ASTM D696 |
| Melting Point, °C | 130 | |

Stability studies were conducted using a pirlimycin aqueous formulation packaged in containers comprising a variety of gamma-irradiation sterilized polymer materials. These polymers represented seven different types of functional monomers. The polymer materials included polystyrene (PS), polycarbonate (PC), polyester (PET), acrylonitrile/butadiene/styrene (ABS), poly(styrene acrylonitrile) (SAN), Nylon 66, LDPE, HDPE and polypropylene (PP). The testing comprised filling the aqueous pirlimycin into the containers, wherein the containers were exposed to gamma irradiation prior to depositing the material into each container.

The expectation based on historical literature was that polymers that undergo more scission would provide a less compatible packaging material than those that undergo more crosslinking. Some known ratios of scission to crosslinking are:

| | |
|---|---|
| Polypropylene | =0.5 (high degree of scission) |
| Polyethylene | =0.3 |
| Polystyrene | =0 (low degree of scission) |

The irradiation doses needed to produce significant damage, i.e., degradation due to scission processes, to these polymers are:

| | |
|---|---|
| Polypropylene | =10 Mrad |
| Polyethylene | =100 Mrad |
| Polystyrene | =1000 Mrad |

Although evolution of volatile organic compounds is less in higher density polyethylenes, LDPE and MDPE can withstand significantly more irradiation than HDPE before experiencing equivalent degradation of physical properties. As an example, LDPE and MDPE can withstand approximately 100 Mrad or more of irradiation before experiencing equivalent elongation under stress as with HDPE irradiated to approximately 10 Mrad.

Crosslinking predominates over scission in polyacrylic esters, polyacrylic acid, polyacrylamide, butadiene-acrylonitrile copolymers and styrene-acrylonitrile copolymers. This is also generally true for aliphatic polyamides, i.e., Nylon 66. Polymers containing aromatic rings as a functional group in the monomer, i.e., polystyrene, polycarbonate and polyester, also generally are more resistant to irradiation-induced degradation than polyolefins, i.e., polyethylene and polypropylene.

Given that scission has been linked with the production of radicals and that scission is related to a degradation of mechanical properties, the relative amount of radiation needed to cause degradation of mechanical properties was used to rank the polymers in their ability to protect product from oxidative degradation. Of the polymers tested, the preliminary ranking was generally as follows, where polypropylene was expected to have the least ability to protect product against oxidative degradation:
PS, PC, PET, ABS, SAN, Nylon 66 > LDPE, HDPE > PP

Unexpectedly, experimental stability studies using the pirlimycin aqueous formulation in gamma-irradiation sterilized packaging produced a much different ranking inconsistent with accepted literature. Based on their ability to protect the pirlimycin aqueous formulation from oxidative degradation, the polymers ranked as follows, where product packaged in SAN suffered the highest level of oxidative degradation.
HDPE > PC > Nylon 66 > PS > PET > PP > LDPE > ABS > SAN
As noted above, differences exist between multiple producers of a given class of polymer. Accordingly, in cases having multiple producers for a given class of polymer, data averages were used to determine ranking.

The foregoing results were supported with similar stability studies performed on an aqueous formulation of another lincosamide antibiotic. Results were further supported with stability studies performed on two formulations of cephalosporin antibiotics, i.e.,
oil-based ceftiofur hydrochloride and ceftiofur crystalline free acid suspensions. In all cases, containers comprised predominantly of polyethylene of density greater than 0.925 g/cc demonstrated acceptably low levels of induced oxidative degradation in the product material.

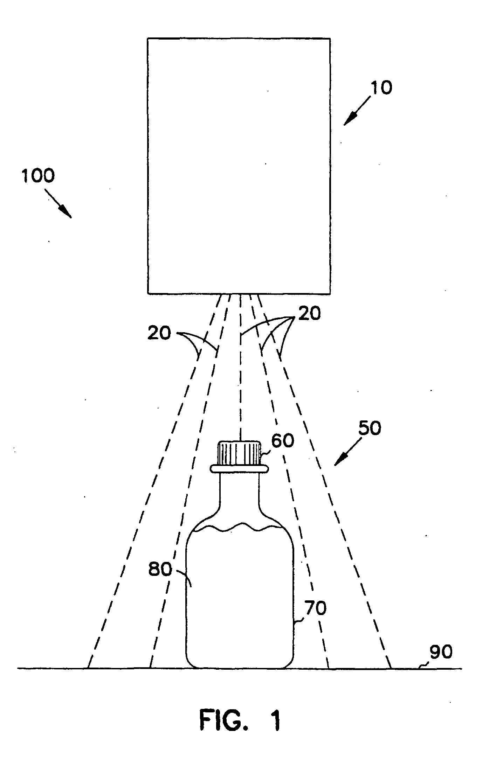

Figure 1 illustrats a known sterilization system which can be used in the present invention. Sterilization system 100 has a radiation source 10 for producing gamma radiation 20 and may also inculdes a conveyor 90 for passing articles through the gamma radiation 20. Radiation source 10 may produce gamma radiation 20 in all directions (not shown) or may focus the gamma radiation 20 to a more localized area as depicted in Figure 1.

Article 50 includes a bottle 70 with a cap 60 surrounding material 80. Bottle 70 and cap 60 may be referred to in combination as a container. Although article 50 is depicted as a bottled material in this embodiment, article 50 can take the form of any three dimensional container surrounding material 80. Furthermore, although material 80 is depicted as a liquid in this embodiment, material 80 can take any physical form, including, but not limited to, solution, solid, gas, powder, granule, tablet, gel, suspension, paste or other physical form. Solutions and suspensions may be aqueous, oil-based or other solvent-based compositions.

At least one component of the container, e.g., cap 60 and bottle 70, contains polyethylene. The polyethylene is of the MDPE or HDPE classification, thus having a density of greater than approximately 0.925 g/cc. A preferred range of polyethylene density is approximately 0.926 to 0.97 g/cc. A more preferred range of polyethylene density is approximately 0.941 to 0.97 g/cc. In one embodiment, the polyethylene is in contact with material 80. In another embodiment, the polyethylene is a predominant constituent of the container.

Article 50 is brought into the gamma radiation 20 on conveyor 90. Article 50 may be moved through the gamma radiation 20 in a continuous fashion, or it may pause within the gamma radiation 20 for a period of time. The exposure for a given intensity of radiation source 10 can be regulated by controlling the speed of the conveyor, or the length of the pause within the gamma radiation 20.

The invention is expected to be most applicable to irradiation dosage levels of up to approximately 100 kGy (10 Mrad). A preferred range of irradiation dosage levels is 15 to 100 kGy (1.5 to 10 Mrad). A more preferred range of irradiation dosage levels is 15 to 60 kGy (1.5 to 6.0 Mrad). A still more preferred range of irradiation dosage levels is 25 to 60 kGy (2.5 to 6.0 Mrad).

While the invention is further applicable to all ambient process temperatures within the processing limits of the polyethylene, a preferred range of ambient process temperatures is above approximately 4°C. A more preferred ambient process temperature is approximately 25°C. The ambient process temperature is the temperature at which the article is exposed to gamma irradiation, and does not reflect any anticipated temperature rise of the article, product material or packaging material due to absorption of the incident radiation.

Material 80 is deposited in the container using packaging techniques well known in the art. As one of ordinary skill in the art will recognize, packaging techniques are dependent upon the nature of the material to be packaged, the nature of the container into which the material is to be packaged, and the quality constraints placed on the finished article. The invention, however, is not dependent upon the packaging technique utilized.

Material 80 may be deposited in the container prior to gamma irradiation as shown in Figure 1. Alternatively, bottle 70 and cap 60 may be exposed to gamma irradiation prior to receiving material 80 in a manner similar to that depicted in Figure 1. Such gamma irradiation of containers or their components is often utilized in conjunction with an aseptic fill operation well understood in the art where it may be desirable to avoid gamma irradiation of an already sterile material. Pre- and post-filling gamma irradiation may also be utilized with the invention. While not generally considered common manufacturing practice, gamma irradiation may further be utilized with the invention during packaging of the material 80 into a container.

In addition, although Figure 1 depicts gamma radiation 20 irradiating article 50 from above, the invention is not dependent upon the angle of incidence of gamma radiation 20. Gamma radiation 20 may irradiate article 50 from any angle as the gamma radiation 20 is expected to pass through article 50. Furthermore, although Figure 1 depicts one radiation source 10 irradiating article 50, the invention is equally applicable to the use of multiple radiation sources 10.

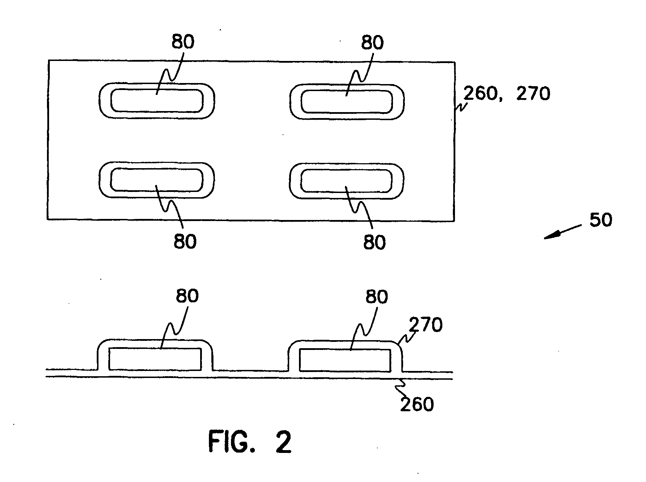

Figure 2 depicts another embodiment of an article 50 in accordance with the invention. Article 50 is depicted as a blister-pack product in this embodiment. Article 50 includes a backing 260 and a blister 270 surrounding material 80. Material 80 is depicted as tablets. Backing 260 and blister 270 may be referred to in combination as a container.

At least one component of the container, i.e., backing 260 and blister 270, contains polyethylene. The polyethylene is of the MDPE or HDPE classification. In one embodiment, the polyethylene is in contact with material 80. In another embodiment. at least one component of the container. i.e.. backing 260 and blister 270. is predominantly polyethylene.

Backing 260 often contains a non-polymer portion, such as a metal foil portion in a composite film commonly used in such packaging configurations. In one embodiment, a polymer portion of backing 260 contains polyethylene. In a further embodiment, a polymer portion of backing 260 is predominantly polyethylene.

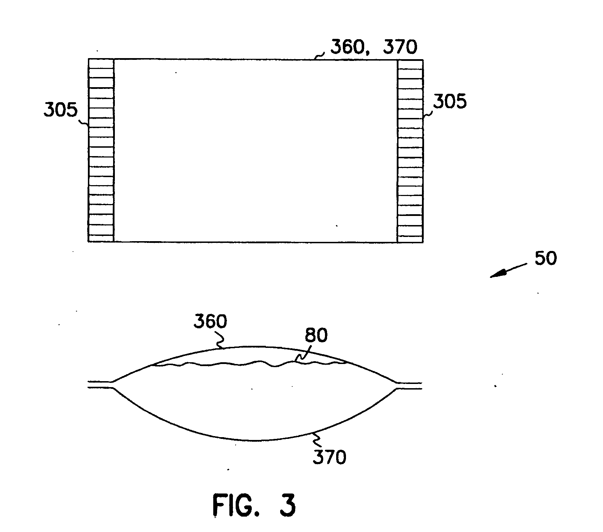

Figure 3 depicts another embodiment of an article 50 in accordance with the invention. Article 50 is depicted as a pouch product in this embodiment. Article 50 includes a first side 360, a second side 370 and seal portions 305 surrounding material 80. Material 80 is depicted as a liquid. Seal portions 305 may be extended further around the perimeter of article 50 depending upon whether article 50 is formed from a polymer tube (as shown), a single sheet of polymer (with seal portions extending around three edges; not shown) or two sheets of polymer (with seal portions extending around four edges; not shown). First side 360 and second side 370 may be referred to in combination as a container.

At least one component of the container, i.e., first side 360 and second side 370, contains polyethylene. The polyethylene is of the MDPE or HDPE classification. In one embodiment, the polyethylene is in contact with material 80. In another embodiment, at least one component of the container, i.e., first side 360 and second side 370, is predominantly polyethylene.

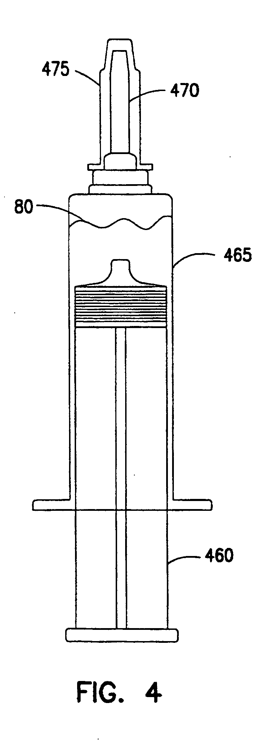

Figure 4 depicts a further embodiment of an article 50 in accordance with the invention. Article 50 is depicted as a syringe product in this embodiment. Article 50 includes a plunger 460, a barrel 465, a cannula 470 and a cap 475 surrounding material 80. Material 80 is depicted as a liquid. Plunger 460, barrel 465, cannula 470 and cap 475 may be referred to in combination as a container.

At least one component of the container, i.e., plunger 460, barrel 465, cannula 470 and cap 475, contains polyethylene. The polyethylene is of the MDPE or HDPE classification. In one embodiment, the polyethylene is in contact with material 80. In one embodiment, at least one component of the container, i.e., plunger 460, barrel 465, cannula 470 and cap 475, is predominantly polyethylene. In another embodiment, barrel 465 is predominantly polyethylene.

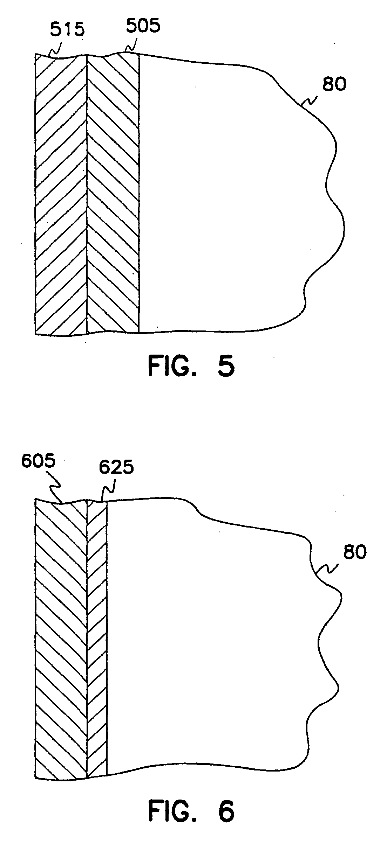

The invention is not limited to the use of containers or their components consisting purely of polyethylene. Additives are commonly found in commercial polyethylenes. Some additives include, but are not limited to, mold-release agents, stabilizers, antioxidants, antirads, compounding agents, lubricants, slip agents, colorants and copolymers. Aside from additives, containers in accordance with the invention may also be composite containers. Two examples of composite containers are depicted in Figures 5 and 6.

Figure 5 depicts a portion of a container wall showing material 80 in contact with a polyethylene layer 505. Polyethylene layer 505 is predominantly polyethylene, but may contain additives as noted above. Polyethylene layer 505 is in contact with layer 515. Layer 515 may be utilized in conjunction with polyethylene layer 505 to improve structural integrity of the composite container, to enhance physical characteristics of the composite container or to reduce the overall cost of the container over one made exclusively of polyethylene. Layer 515 may be of any composition consonant with the aforementioned goals. Common compositions include metals for improved structural integrity, glass for impermeability and fiberboard for reduced cost.

Figure 6 depicts a portion of a container wall showing material 80 in contact with a semi-permeable layer 625. Semi-permeable layer 625 is in contact with polyethylene layer 605. Polyethylene layer 605 is predominantly polyethylene, but may contain additives as noted above. Semi-permeable layer 625 protects polyethylene layer 605 from physical contact with material 80, but is permeable to irradiation-induced radicals in polyethylene layer 605 such that the irradiation-induced radicals are free to migrate through semi-permeable layer 625 to a surface of material 80. Accordingly, polyethylene layer 605 is in contact with material 80 as defined above.

### Conclusion

Methods of packaging oxidation-sensitive medicinal substances are thus disclosed. In the foregoing specification this invention has been described in relation to certain embodiments thereof, and many details have been set forth for purposes of illustration. As an example, the invention is suitable for a wide variety of containers, including, but not limited to, bottles, vials, mastitis syringes, prescription syringes, ampules, pouches, blister packs, cylinders, tubes, drums, pails, canisters and more. Therefore, it is manifestly intended that this invention be limited only by the claims.

## Claims

1. A method of packaging an oxidation-sensitive medicinal substance used to prevent or treat disease, injury or pain, comprising:
depositing the medicinal substance in a container, wherein the container comprises polyethylene having a density of greater than 0.925 g/cc; and
before, during or after the depositing, exposing the container to gamma irradiation.

2. A method according to claim 1, wherein the medicinal substance is an anti-infective.

3. A method according to claim 2, wherein the anti-infective is an antibiotic.

4. A method according to claim 3, wherein the antibiotic is selected from cephalosporins, lincosamides, quinolones, oxazolidinones, tetracyclines, penicillin and penicillin derivatives.

5. A method according to claim 3, wherein the antibiotic is selected from pirlimycin, ceftiofur, lincomycin, neomycin, penicillin G and novobiocin.

6. A method according to any preceding claim, wherein the exposing is at an ambient temperature above 4°C.

7. A method according to claim 6, wherein the ambient temperature is approximately 25°C.

8. A method according to any preceding claim, wherein the polyethylene is in contact with the medicinal substance after depositing the medicinal substance in the container.

9. A method according to any preceding claim, wherein the gamma irradiation is at a dosage of up to 100 kGy.

10. A method according to claim 9, wherein the dosage is 15 to 60 kGy.

11. A method according to claim 9, wherein the dosage is 25 to 60 kGy.

12. A method according to any preceding claim, wherein the polyethylene has a density of 0.926 to 0.97 g/cc.

13. A method according to claim 12, wherein the polyethylene has a density of 0.941 to 0.97 g/cc.

## Patentansprüche

1. Verfahren der Verpackung eines oxidationsempfindlichen Arzneimittels, das zur Prophylaxe oder Behandlung einer Erkrankung, Verletzung oder von Schmerzen verwendet wird, das umfasst:
das Plazieren des Arzneimittels in einen Behälter, wobei der Behälter Polyethylen mit einer Dichte von größer als 0,925 g/cm³ umfasst; und
das Bestrahlen des Behälters mit Gamma-Strahlung vor, während oder nach dem Plazieren.

2. Verfahren nach Anspruch 1, wobei das Arzneimittel ein Antiinfektiosum ist.

3. Verfahren nach Anspruch 2, wobei das Antiinfektiosum ein Antibiotikum ist.

4. Verfahren nach Anspruch 3, wobei das Antibiotikum aus Cephalosporinen, Lincosamiden, Chinolonen, Oxazolidinonen, Tetracyclinen, Penicillin und Penicillinderivaten ausgewählt ist.

5. Verfahren nach Anspruch 3, wobei das Antibiotikum aus Pirlimycin, Ceftiofur, Lincomycin, Neomycin, Penicillin G und Novobiocin ausgewählt ist.

6. Verfahren nach einem der vorhergehenden Ansprüche, wobei die Bestrahlung bei Umgebungstemperatur über 4 °C erfolgt.

7. Verfahren nach Anspruch 6, wobei die Umgebungstemperatur etwa 25 °C beträgt.

8. Verfahren nach einem der vorhergehenden Ansprüche, wobei das Polyethylen nach dem Plazieren des Arzneimittels in dem Behälter mit dem Arzneimittel in Kontakt ist.

9. Verfahren nach einem der vorhergehenden Ansprüche, wobei die Gamma-Bestrahlung mit einer Dosis von bis zu 100 kGy erfolgt.

10. Verfahren nach Anspruch 9, wobei die Dosis 15 - 60 kGy beträgt.

11. Verfahren nach Anspruch 9, wobei die Dosis 25 - 60 kGy beträgt.

12. Verfahren nach einem der vorhergehenden Ansprüche, wobei das Polyethylen eine Dichte von 0,926 - 0,97 g/cm³ aufweist.

13. Verfahren nach Anspruch 12, wobei das Polyethylen eine Dichte von 0,941 - 0,97 g/cm³ aufweist.

## Revendications

1. Procédé pour l'emballage d'une substance médicamenteuse sensible à l'oxydation utilisée pour la prévention ou le traitement d'une maladie, d'une lésion ou de la douleur, comprenant les étapes consistant :
à déposer la substance médicamenteuse dans un récipient, le récipient comprenant un polyéthylène ayant une masse volumique supérieure à 0,925 g/cm³ ; et
avant, pendant ou après le dépôt, à exposer le récipient à une irradiation avec des rayons gamma.

2. Procédé suivant la revendication 1, dans lequel la substance médicamenteuse est un agent anti-infectieux.

3. Procédé suivant la revendication 2, dans lequel l'agent anti-infectieux est un antibiotique.

4. Procédé suivant la revendication 3, dans lequel l'antibiotique est choisi entre des céphalosporines, des lincosamides, des quinolones, des oxazolidinones, des tétracyclines, la pénicilline et des dérivés de pénicilline.

5. Procédé suivant la revendication 3, dans lequel l'antibiotique est choisi entre la pirlimycine, le ceftiofur, la lincomycine, la néomycine, la pénicilline G et la novobiocine.

6. Procédé suivant l'une quelconque des revendications précédentes, dans lequel l'exposition est effectuée à une température ambiante supérieure à 4°C.

7. Procédé suivant la revendication 6, dans lequel la température ambiante est approximativement égale à 25°C.

8. Procédé suivant l'une quelconque des revendications précédentes, dans lequel le polyéthylène est en contact avec la substance médicamenteuse après avoir déposé la substance médicamenteuse dans le récipient.

9. Procédé suivant l'une quelconque des revendications précédentes, dans lequel l'irradiation avec des rayons gamma est effectuée à une dose allant jusqu'à 100 kGy.

10. Procédé suivant la revendication 9, dans lequel la dose est comprise dans l'intervalle de 15 à 60 kGy.

11. Procédé suivant la revendication 9, dans lequel la dose est comprise dans l'intervalle de 25 à 60 kGy.

12. Procédé suivant l'une quelconque des revendications précédentes, dans lequel le polyéthylène a une masse volumique comprise dans l'intervalle de 0,926 à 0,97 g/cm³.

13. Procédé suivant la revendication 12, dans lequel le polyéthylène a une masse volumique comprise dans l'intervalle de 0,941 à 0,97 g/cm³.
